# EUROPEAN PATENT SPECIFICATION

(11) **EP 0 979 657 B1**
(45) Date of publication and mention of the grant of the patent: **15.02.2006**
(21) Application number: 99115761.1
(22) Date of filing: 10.08.1999
(51) Int. Cl.: A61L 2/00, A23L 3/3454

(54) **Method and apparatus for continuous flow reduction of microbial activity in a liquid product using pressurized carbon dioxide**
Verfahren und Apparat zur Reduktion von mikrobieller Aktivität in einem kontinuierlichen Strom eines flüssigen Produktes mittels Kohlendioxyd unter Hochdruck
Procédé et appareil pour réduire l'activité microbienne d'un produit liquide en fluide continu au moyen de dioxyde de carbone pressurisé

(30) Priority: 10.08.1998 US 95967 P; 20.05.1999 US 314945
(43) Date of publication of application: 16.02.2000
(73) Proprietor: University of Florida Research Foundation, Inc., Gainesville, FL 32611-5500 (US)
(72) Inventor: Wildasin, Richard E., Lockport, Illinois 60441 (US); Forbes, James, Lockport, Illinois 60441 (US); Robey, Raymond J., Naperville, Illinois 60564 (US)
(74) Representative: Schwan, Gerhard

(56) References cited:
- US-A- 3 477 856
- US-A- 5 393 547
- US-A- 5 704 276
- PATENT ABSTRACTS OF JAPAN vol. 015, no. 150, 16 April 1991 (1991-04-16) & JP 03 027268 A (SHOKUHIN SANGYO HAI SEPAREESHIYON SYST GIJUTSU KENKYU KUMIAI), 5 February 1991 (1991-02-05)
- DATABASE FSTA [Online] INTERNATIONAL FOOD INFORMATION SERVICE (IFIS), FRANFURT/MAIN, DE ARREOLA ET AL.: "Supercritical carbon dioxide effects on some quality attributes of single strength orange juice" XP002121723 & JOURNAL OF FOOD SCIENCE., vol. 56, no. 4, 1991, pages 1030-1033, INSTITUTE OF FOOD TECHNOLOGISTS. CHICAGO., US ISSN: 0022-1147
- DATABASE FSTA [Online] INTERNATIONAL FOOD INFORMATION SERVICE (IFIS), FRANFURT/MAIN, DE ISHIKAWA ET AL.: "Sterilization of microorganisms by the supercritical carbon dioxide micro-bubble method" XP002121722 & BIOSCIENCE, BIOTECHNOLOGY AND BIOCHEMISTRY, vol. 59, no. 10, 1995, pages 1949-1950, XX, XX ISSN: 0916-8451
- DATABASE FSTA [Online] INTERNATIONAL FOOD INFORMATION SERVICE (IFIS), FRANFURT/MAIN, DE KAMIHIRA: "Sterilization of microorganisms with supercritical carbon dioxide" XP002121724 & AGRICULTURAL AND BIOLOGICAL CHEMISTRY., vol. 51, no. 2, 1987, pages 407-412, JAPAN SOC. FOR BIOSCIENCE, BIOTECHNOLOGY AND AGROCHEM. TOKYO., JP ISSN: 0002-1369

## Description

### FIELD OF THE INVENTION

This invention relates to a method and apparatus for the processing of liquids to reduce microbial and/or enzymatic activity therein and, more particularly, to the use of pressurized carbon dioxide to achieve reductions of microbial and/or enzymatic activity.

### BACKGROUND OF THE INVENTION

There are many methods for improving the shelf life of liquid products such as orange juice, apple juice, milk, latex paints, peanut butter, soup, etc.

Commercially, thermal methods such as pasteurization are the predominant methods used to improve the shelf life of liquid foods. Ultra-high pressure treatment is also used for liquid foods, but much less frequently.

In high pressure treatment facilities, fluids containing microbial contamination are pressurized hydrostatically to kill the majority of the bacteria. In such systems, pressures are created which equal or exceed 2068 bar (30,000 psia). Such hydrostatic treatment, however, does not destroy enzymes, is unsafe because of the very high pressures, is a lengthy process, is batch rather than continuous, and is expensive due to the high capital costs of the required equipment.

Other methods for shelf-life extension of liquids include nuclear irradiation, ultra-violet exposure and application of microwaves. These treatments are expensive and not widely used commercially at present.

High pressure homogenization has been used to increase the shelf life of orange juice and other single-strength citrus juices as described in U.S. Patent 5,232,726 to Clark et al. It is disclosed that a citrus juice being processed is subjected to a high pressure of about 1034 bar (15,000 psia), with the result being a significant reduction in biological activity in the juice.

Carbon dioxide has been used to inactivate enzymes in food and reduce microbial populations in fruit juices as described in U.S. Patent 5,393,547 to Balaban et al. Balaban et al. describe a method for inactivating enzymes in liquid food products wherein the food is exposed to pressurized carbon dioxide which, in turn, produces a carbonic acid solution with a pH that is sufficiently low to irreversibly inactivate enzymes in the liquid food. The Balaban et al. method is indicated as being applicable to either batch mode or continuous flow mode processing of food. Balaban et al. further indicate that supercritical carbon dioxide is introduced at a rate sufficient to allow enough thereof to dissolve in the food to inactivate the enzymes. After enzymatic inactivation, the food flows to a section where pressure is reduced and the released carbon dioxide may be recycled for repeat usage.

U.S. Patent 5,704,276 to Osajima et al. describes a method for continuous deactivation of enzymes in liquid foodstuffs, using a supercritical form of carbon dioxide. Osajima et al. indicate that the density of the supercritical fluid is less than that of the liquid food and that the supercritical carbon dioxide is injected continuously into the liquid food and is separated therefrom in a later stage of the process. Osajima et al. also indicate that their process deodorizes the liquid food and removes volatile components.

Arreola et al. in "Effect of Supercritical Carbon Dioxide on Microbial Populations in Single Strength Orange Juice", Journal of Food Quality, Volume 14 (1991), pp. 275-284, describe the effect of supercritical carbon dioxide on microbial populations in orange juice. Using a batch process, Arreola et al. concluded that high pressure carbon dioxide treatment resulted in microbial reduction in single strength orange juice, even at low temperatures. Further, they conclude that a combination of high pressure, and shear forces to which the orange juice is subjected during depressurization and lower pH due to temporary formation of carbonic acid may have further inhibitory effects on the normal flora within orange juice. During the processing described in this paper, the minimum temperature utilized was 35°C.

It is an object of this invention to provide an improved method and apparatus for reducing microbial and/or enzymatic activity in liquid products.

It is a further object of this invention to provide a method and apparatus for reducing microbial and/or enzymatic activity in liquid products using pressurized carbon dioxide, wherein the processing temperature to which the liquid is subjected does not deleteriously affect the liquid products.

It is yet another object of this invention to provide a continuous flow method and apparatus for reducing microbial and/or enzymatic activity in liquid products using pressurized carbon dioxide.

### SUMMARY OF THE INVENTION

A continuous method using a pressurized flow of carbon dioxide is described for the reduction of microorganisms present in the liquid product and/or the inactivation of one or more enzymes in a pressurized flow of the liquid product. The pressure in the flow regions are maintained at a level which is sufficient to keep the carbon dioxide in dense phase, but at a temperature which does not freeze the liquid product. The pressurized mixture of the carbon dioxide and liquid flows through a reaction zone for a sufficient time to reduce harmful microorganisms and inactivate undesirable enzymes and then enters a plurality of expansion stages wherein the pressure of the mixture flow is decreased sufficiently to allow the separation of carbon dioxide from the liquid product. Heat is applied in at least some of the expansion stages to prevent a cooling of the mixture flow to the freezing point of the liquid product. Heat may be applied to prevent the freezing of the liquid product to control the temperature so that it does not exceed a temperature at which deleterious effects are experienced. (Freezing and excessive high temperature can have negative effects on the juice quality. Temperatures over 40°C begin to degrade the product.)

The present invention is contemplated for use with any liquid that may be transported through a conduit, including for example, beverage products such as juices and milk, semi-liquid foods such as mayonnaise, salad dressings, soup and cottage cheese, and other liquids such as paint and sterile injectibles.

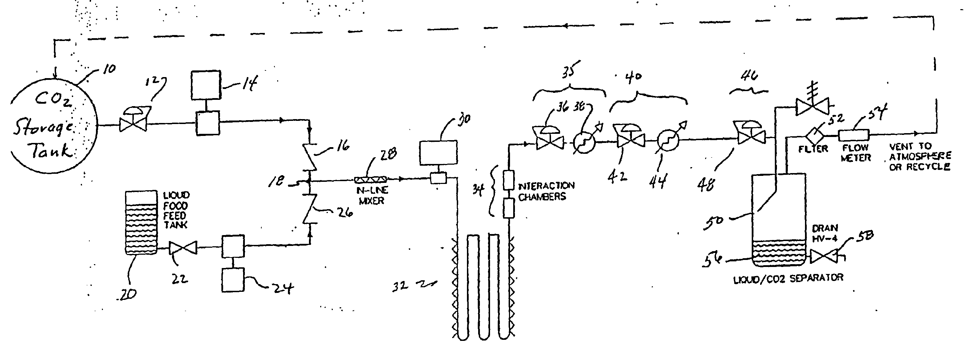

### BRIEF DESCRIPTION OF THE DRAWING

The figure is a schematic flow diagram of apparatus which performs the method of the invention.

### DETAILED DESCRIPTION OF THE INVENTION

Referring to the figure, pressurized carbon dioxide is fed from carbon dioxide supply 10 through optional pressure regulator 12 to a pump 14 which increases the pressure of the carbon dioxide flow and then feeds it through a check valve 16 to a juncture 18. The carbon dioxide is pressurized at pump 14 to prevent any boiling of the dense phase carbon dioxide during later stages of the process.

In similar fashion, liquid product is fed from a liquid product feed tank 20 through a valve 22 to a pump 24. Pump 24 raises the feed pressure of the liquid product to the same level as that of the dense phase carbon dioxide exiting from pump 14. The pressurized liquid product feed passes through check valve 26 to juncture 18 where it combines with the pressurized flow of carbon dioxide. The mixture of the liquid product and carbon dioxide then passes to an in-line mixer 28 which essentially comprises a heavily baffled conduit that thoroughly mixes the carbon dioxide and liquid product streams. Of course, other mixers may be employed which achieve a desired level of liquid product/carbon dioxide mixing. The liquid mixture exits from in-line mixer 28 and is further pressurized by the action of pump 30 to a process pressure.

Depending upon the specific liquid product feed, the process pressure will vary accordingly. It is preferred that the process pressure be within the range of 20.7 bar to 1379 bar (300 psia to 20,000 psia). If orange juice is being processed as a liquid food, a preferred range of pressure is about 120.7 bar to about 151.7 bar (about 1750 psia to about 2200 psia).

Once the liquid mixture exits from pump 30, it enters a reaction zone 32 that is of suitable size and length to provide sufficient contact (or residence) time for the carbon dioxide and liquid product to interact in a manner which reduces microorganisms and/or inactivates undesirable enzymes present in the liquid product. The selected residence time will depend on the liquid product to be processed and its flowrate, as well as the size and length of the reaction zone. It is preferred that the reaction zone residence time is in the range of about 1.0 to about 15.0 minutes.

For example, for processing orange juice, at a flowrate of 20-200 ml/min in a reaction zone having a length of about 6.10 m (20 feet) and tubing size of about 7.9mm inner diameter (I.D.), the preferred residence time is about 1.5 to 13.0 minutes, and more preferably about 3.0 minutes of residence time.

As the liquid mixture stream exits from reaction zone 32, it enters one or more interaction chambers 34 (optional) wherein high shear forces are applied which enable a rupture of microbial cell walls in the liquid mixture. Such action enables a further reduction of the microbial populations in the liquid mixture. High shear interaction chambers that are suitable for inclusion in this process are manufactured by the Microfluidics International Corp., Newton, Massachusetts.

At this stage, the pressurized carbon dioxide/liquid product mixture must be depressurized in such a fashion as to avoid freezing the liquid product (due to the Joule-Thompson cooling effect of the expansion of the carbon dioxide). If the pressure is lowered to ambient in one or two stages, very large heat exchange or application of supplemental heat is required. If too much heat is added to the mixture, damage will occur to the liquid product, either in its flavor characteristics or its composition. Also, important volatiles such as flavor components may be carried away. Accordingly, it has been found that substantial care must be taken during the depressurization action to maintain the liquid mixture within two boundaries. The lower boundary is the freezing point of the liquid mixture and the upper boundary point is the maximum temperature to which the liquid product can be subjected, without damage to the product.

In the case of orange juice, the maximum temperature is about 60°C and the minimum temperature is about 0°C. Accordingly, when choosing a pressure reduction scheme, a pressure/enthalpy chart for carbon dioxide is followed to determine the optimum pressure and heating temperature needed for plural pressure reduction stages, while keeping (in this example) the orange juice at a temperature between that which will injure its flavor and its freezing point. It has been determined that at least two stages of depressurization are required, but it is preferred that there be at least three stages.

Returning to the figure, the first depressurization stage includes a pressure control device 36, such as a back pressure regulator, followed by a heat exchanger 38. Assuming that the liquid product being processed is orange juice and that the process pressure within reaction zone 32 and interaction chamber 34 is about 137.9 bar (2,000 psia), a first depressurization stage 35 reduces the pressure of the liquid mixture to approximately 42.4 bar (600 psig) and applies sufficient heat through heat exchanger 38 to maintain the liquid mixture at about 30°C.

A second depressurization stage 40 includes a pressure control device 42 and heat exchanger 44 which, in combination, reduce the pressure of the liquid mixture to about 17.2 bar (250 psia) and maintains its temperature at approximately 30°C. A final stage depressurizer 46 includes only a pressure control device 48 to reduce the pressure of the liquid mixture to the point where the dense phase carbon dioxide will vaporize and may be separated from the liquid products while minimizing loss of important volatile components. In the embodiment shown in the figure, no heat exchanger is required subsequent to pressure control device 48, however, one may be provided, if required, to maintain the liquid mixture within the required temperature range.

As the liquid mixture exits from pressure control device 48, it enters a liquid product/carbon dioxide separator vessel 50 or other collection device at reduced pressure. There, the carbon dioxide vapor separates from the liquid product, is captured and passed through a filter 52, flow meter 54 (if desired) and is either vented to atmosphere or is passed through a pressurization stage (not shown) for recycling back to carbon dioxide supply 10. The liquid product pool 56 may then be drained through valve 58 for subsequent processing and/or use.

It is to be understood, that the continuous process method shown in the figure is made practical by the multiple depressurization stages which enable the liquid mixture to be maintained within the aforementioned temperature boundaries. As a result, a continuous process for reduction of microbial and/or enzymatic activity is achieved while overcoming the principal problem of the prior art, i.e., batch processing which is an uneconomic and undesired processing procedure in a commercial environment.

If the carbon dioxide gas is to be recycled, it is preferred that it be passed through a coalescing filter to remove droplets of the processed liquid product. Thereafter, the gas is recondensed to the liquid state by passage through a condensing heat exchanger. Further, to assure removal of the dissolved carbon dioxide in the processed liquid product, a liquid product/carbon dioxide separator downstream from separator tank 50 may include means for deaeration.

The resultant gas, remaining after processing, may carry additional valuable aromas and/or flavors. To recover or remove such aromas or flavors, a method such as condensation or absorption may be utilized.

It should be understood that the foregoing description is only illustrative of the invention. Various alternatives and modifications can be devised by those skilled in the art without departing from the invention.

## Claims

1. A continuous method for reducing microorganisms in a liquid product, said method comprising the steps of:
a) combining a pressurized flow of said liquid product with a flow of pressurized liquefied carbon dioxide to create a pressurized mixture in a flow state, said carbon dioxide at a pressure sufficient to maintain it in a liquid state and at a temperature which does not freeze said liquid product;
b) flowing said pressurized mixture through a reaction zone for a sufficient time to reduce microorganisms in said liquid product;
c) feeding said pressurized mixture from said reaction zone through plural expansion stages wherein the pressure of said mixture flow is decreased to vaporize the liquefied carbon dioxide in said mixture flow; and
d) applying heat in at least some of said expansion stages to said mixture flow to prevent a cooling of said carbon dioxide from causing a freezing of said liquid product.

2. The continuous method as recited in claim 1, wherein step d) maintains a temperature of said mixture within a range between a freezing temperature of said liquid product and about 60°C.

3. The continuous method as recited in claim 1, wherein step c) feeds said mixture flow through two or more expansion stages to vaporize said liquefied carbon dioxide.

4. The continuous method as recited in claim 1, wherein step a) feeds said pressurized flow of said mixture in said reaction zone at a pressure within a range of about 20.7 bar to about 1379 bar (about 300 psia to about 20,000 psia).

5. The continuous method as recited in claim 1, wherein step b) maintains said pressurized flow of said mixture in said reaction zone for a duration of from about 5 seconds to about 30 minutes.

6. The continuous method as recited in claim 1, wherein said liquid product is a food product and said method inactivates one or more undesirable enzymes.

7. The continuous method as recited in claim 1, wherein said liquid product is a liquid juice product; wherein said method is for inactivating one or more undesirable enzymes in said liquid juice product; wherein in step b) the contact time is about 1.0 to about 15 minutes to inactivate said one or more undesirable enzymes; and wherein in step c) the pressure of said mixture flow is decreased to about 137.9 bar (2,000 psia).

8. The continuous method as recited in claim 7, wherein the juice is a vegetable or fruit juice and wherein the contact time in step b) is about 1.5 to about 13 minutes.

9. The continuous method as recited in claim 7, wherein step d) maintains a temperature of said mixture within a range between a freezing temperature of said liquid juice product and about 30°C.

10. The continuous method as recited in claim 8, wherein said juice is orange juice, said contact time is about 3.0 minutes, and wherein step d) maintains a temperature of said mixture at about 30°C.

## Patentansprüche

1. Kontinuierliches Verfahren zum Verringern von Mikroorganismen in einem flüssigen Produkt, wobei im Zuge des Verfahrens:
a) ein unter Druck stehender Strom des flüssigen Produkts mit einem Strom von unter Druck stehendem, verflüssigtem Kohlendioxyd kombiniert wird, um ein unter Druck stehendes Gemisch in einem Strömungszustand zu erzeugen, wobei das Kohlendioxyd unter einem Druck steht, der hinreichend ist, um es in einem flüssigen Zustand zu halten, und sich bei einer Temperatur befindet, bei welcher das flüssige Produkt nicht gefriert;
b) das unter Druck stehende Gemisch über einen Zeitraum durch eine Reaktionszone strömt, der hinreichend ist, um Mikroorganismen in dem flüssigen Produkt zu verringern;
c) das unter Druck stehende Gemisch von der Reaktionszone durch mehrere Expansionsstufen geleitet wird, worin der Druck des strömenden Gemisches verringert wird, um das verflüssigte Kohlendioxyd in dem strömenden Gemisch zu verdampfen; und
d) in mindestens einigen der Expansionsstufen das strömende Gemisch mit Wärme beaufschlagt wird, um zu verhindern, dass ein Abkühlen des Kohlendioxyds ein Frieren des flüssigen Produkts bewirkt.

2. Kontinuierliches Verfahren gemäß Anspruch 1, wobei in Schritt d) das Gemisch bei einer Temperatur in einem Bereich zwischen dem Gefrierpunkt des flüssigen Produkts und etwa 60°C gehalten wird.

3. Kontinuierliches Verfahren gemäß Anspruch 1, wobei in Schritt c) das strömende Gemisch durch zwei oder mehr Expansionsstufen geleitet wird, um das verflüssigte Kohlendioxyd zu verdampfen.

4. Kontinuierliches Verfahren gemäß Anspruch 1, wobei in Schritt a) der unter Druck stehende Strom des Gemischs der Reaktionszone bei einem Druck im Bereich von etwa 20,7 bar bis etwa 1.379 bar (etwa 300 psia bis etwa 20.000 psia) zugeführt wird.

5. Kontinuierliches Verfahren gemäß Anspruch 1, wobei in Schritt b) der unter Druck stehende Strom des Gemischs in der Reaktionszone für einen Zeitraum von mindestens 5 Sekunden bis etwa 30 Minuten gehalten wird.

6. Kontinuierliches Verfahren gemäß Anspruch 1, wobei es sich bei dem flüssigen Produkt um ein Nahrungsmittelprodukt handelt und bei dem Verfahren mindestens ein nicht erwünschtes Enzym inaktiviert wird.

7. Kontinuierliches Verfahren gemäß Anspruch 1, wobei es sich bei dem flüssigen Produkt um ein flüssiges Saftprodukt handelt, wobei das Verfahren dafür ausgelegt ist, mindestens ein unerwünschtes Enzym in dem flüssigen Saftprodukt zu inaktivieren, wobei in Schritt b) die Kontaktzeit etwa 1,0 bis etwa 15 Minuten beträgt, um das mindestens eine unerwünschte Enzym zu inaktivieren; und wobei in Schritt c) der Druck des strömenden Gemischs auf etwa 137,9 bar (2.000 psia) verringert wird.

8. Kontinuierliches Verfahren gemäß Anspruch 7, wobei es sich bei dem Saft um einen Gemüse- oder Fruchtsaft handelt und wobei die Kontaktzeit im Schritt b) etwa 1,5 bis etwa 13 Minuten beträgt.

9. Kontinuierliches Verfahren gemäß Anspruch 7, wobei in Schritt d) eine Temperatur des Gemischs in einem Bereich zwischen dem Gefrierpunkt des flüssigen Saftprodukts und etwa 30°C aufrecht erhalten wird.

10. Kontinuierliches Verfahren gemäß Anspruch 7, wobei es sich bei dem Saft um Orangensaft handelt, die Kontaktzeit etwa 3,0 Minuten beträgt und in Schritt d) eine Temperatur des Gemischs von etwa 30°C aufrecht erhalten wird.

## Revendications

1. Procédé de réduction en continu de micro-organismes dans un produit liquide, ledit procédé comprenant les étapes de :
a) combinaison d'un flux sous pression dudit produit liquide et d'un flux de gaz carbonique liquéfié et sous pression, pour créer un mélange sous pression à l'état d'écoulement, ledit gaz carbonique étant à une pression suffisante pour le maintenir à l'état liquide, et à une température non susceptible de solidifier par congélation ledit produit liquide ;
b) écoulement dudit mélange sous pression à travers une zone de réaction pendant une durée suffisante pour réduire des micro-organismes présents dans ledit produit liquide ;
c) passage dudit mélange sous pression, à partir de ladite zone de réaction, à travers une pluralité d'étages de détente, où la pression dudit écoulement de mélange est diminuée afin de vaporiser le gaz carbonique liquéfié, présent dans ledit écoulement de mélange ; et
d) application de chaleur, dans au moins certains desdits étages de détente, audit écoulement de mélange afin d'éviter qu'un refroidissement dudit gaz carbonique ne provoque une solidification par congélation dudit produit liquide.

2. Procédé continu selon la revendication 1, dans lequel l'étape d) maintient une température dudit mélange dans une plage comprise entre une température de congélation dudit produit liquide et environ 60°C.

3. Procédé continu selon la revendication 1, dans lequel l'étape c) fait passer ledit écoulement de mélange à travers deux ou davantage d'étages de détente, afin de vaporiser ledit gaz carbonique liquéfié.

4. Procédé continu selon la revendication 1, dans lequel l'étape a) amène ledit écoulement sous pression dudit mélange, dans ladite zone de réaction, à une pression comprise dans une plage d'environ 20,7 bars à environ 1379 bars (d'environ 300 psia (pression absolue en livres par pouce carré) à environ 20 000 psia).

5. Procédé continu selon la revendication 1, dans lequel l'étape b) maintient ledit écoulement sous pression dudit mélange, dans ladite zone de réaction, pendant une durée comprise entre environ 5 secondes et environ 30 minutes.

6. Procédé continu selon la revendication 1, dans lequel ledit produit liquide est un produit alimentaire, et ledit procédé inactive une ou plusieurs enzymes indésirables.

7. Procédé continu selon la revendication 1, dans lequel ledit produit liquide est un produit formant jus liquide ; dans lequel ledit procédé est destiné à inactiver une ou plusieurs enzymes indésirables dans ledit produit formant jus liquide ; dans lequel, dans l'étape b), la durée de contact est d'environ 1,0 à environ 15 minutes pour inactiver ladite ou lesdites enzymes indésirables ; et dans lequel, dans l'étape c), la pression dudit écoulement de mélange est diminuée jusqu'à environ 137,9 bars (2000 psia).

8. Procédé continu selon la revendication 7, dans lequel le jus est un jus de légume ou de fruit, et dans lequel la durée de contact, dans l'étape b), est d'environ 1,5 à environ 13 minutes.

9. Procédé continu selon la revendication 7, dans lequel l'étape d) maintient une température dudit mélange dans une plage comprise entre une température de solidification par congélation dudit produit formant jus liquide, et environ 30°C.

10. Procédé continu selon la revendication 8, dans lequel ledit jus est du jus d'orange, ladite durée de contact est d'environ 3,0 minutes, et dans l'étape d) maintient une température dudit mélange à environ 30°C.
